# EUROPEAN PATENT APPLICATION

(11) **EP 4 678 735 A1**
(43) Date of publication of application: **14.01.2026**
(21) Application number: 24187138.3
(22) Date of filing: 08.07.2024
(51) Int. Cl.: C12N 5/00, C12N 5/073, C12N 5/074, C12N 5/078, C12N 5/0789, C12M 1/12, A61K 35/545, C12N 5/0735, A61K 35/12

(54) **METHOD OF HIGH-DENSITY CELL CULTURE AND CELLS THEREOF**

(71) Applicant: HemostOD SA, 1025 Saint-Sulpice (CH)
(72) Inventor: Dahan, Elodie, 1004 Lausanne (CH)
(74) Representative: DTS Patent- und Rechtsanwälte PartmbB

(57) **Abstract**

The present invention provides a method of high-density cell culture comprising at least the following steps: S1: obtain hydrogel microcapsules through membrane emulsification; S2: encapsulate a selected number of cells per each obtained hydrogel microcapsule, and S3: culture the encapsulated cells in a cell culture medium. The invention further refers to Human Pluripotent Stem Cells (hPSCs) obtained through the above-described method, in particular for use in medicine, more in particular for use in a method of *in vitro* generation of mature megakaryocytes.

## Description

The present invention belongs to the technical field of cell culture, in particular high-density cell culture.

Preferably, the cultured cells are Stem Cells or Stem-Cell-Derived-cells.

Stem Cells are categorized into Pluripotent Stem Cells (PSCs), including Embryonic Stem Cells (ESCs) or Induced Pluripotent Stem Cells (iPSCs), and Non-Embryonic or Somatic Stem Cells, commonly called adult stem cells (ASCs).

While ESCs and iPSCs cells can become all cell types, ASCs are limited to differentiate towards their tissue of origin.

The need of Stem Cells and Stem-Cell-Derived Cells with consistent quality is increasingly needed in biomedical applications, e.g., for research studies, therapeutic strategies, or disease modeling (Grompe 2002) (Prochazkova et al. 2015) (Passier et Mummery 2003) **[1], [2], [3].**

Nevertheless, growing PSCs and ASCs in large numbers, preferably with limited costs, is still a challenge.

This challenge is exemplified, for instance, by the *in vitro* production of platelet concentrate derived from human PSCs (hPSCs).

hPSCs can be inter alia used for deriving megakaryocyte progenitor cells.

Megakaryocyte progenitor cells derived from hPSCs can be used to obtain mature megakaryocytes (MKs) for use in techniques for *in vitro* production of engineered blood platelets, e.g., for medical use.

For instance, blood platelets so obtained can be used for the preparation of pharmaceutical compositions for treatment, e.g., target-specific treatment, of a variety of diseases including cancer, acute inflammation, chronic inflammation, bleeding disorders, coagulation disorders, and/or liver diseases.

To cover the specific needs of Stem Cells and maintain optimal proliferation, the used stem cell culture medium shall include small molecules, cytokines, and other growth factors.

These complex media recipes are costly.

By maximizing cell culture density (i.e., the number of cells per unit volume), the culture costs can be correspondingly minimized, or at least significantly reduced.

Sugimoto et al. published, in 2022, results of a clinical trial, describing the culture of 16*109 megakaryocyte progenitors in 4 x 8 L reactors, corresponding to a concentration of 0.5*10⁶ cells per mL, to produce 3 platelet concentrates of a concentration of 0.1x 10¹¹, 0.3*10¹¹ and 1.0*10¹¹ PLT per platelet concentrate (Sugimoto et al. 2022) **[4].**

This large amount of medium is apparently incompatible with platelet concentrate production on a clinically relevant scale.

In particular, to produce 1 PLT concentrate of 3*10¹¹ PLTs, 3.10⁹ MK progenitors must be cultured, considering a yield of 100 PLTs per MK.

Each year, more than 8 million platelet concentrates are transfused in patients worldwide.

Therefore, to cover only a quarter of the demand, more than 10¹⁵ MKs per year are needed.

With current concentration (0.5*10⁶ per mL), this level of production is unrealistic, representing multiple batches of 10,000 L of medium.

Bioreactors are promising platforms for large scale 3D culture of Stem Cells.

To ensure that all cells receive the necessary amount of oxygen and nutrients, they must be constantly and vigorously stirred.

PSCs and ASCs are sensitive to shear stresses induced, for example, by constant agitation, which renders cell culture on a large scale particularly difficult, e.g., when compared to Chinese Hamster Ovary (CHO) cell culture, commonly carried out in up to 10,000 L reactors.

hPSCs tend to form small clusters, helping with cell survival and cell growth, as well as promoting cell-cell interactions.

However, 3D suspension cultures tend to exacerbate this phenomenon, so that cell clusters tend to fuse and create larger agglomerates.

Cells that are located in the center of these agglomerates become less accessible, and thus receive a lower intake of cytokines and O₂. This may hinder cell growth, sometimes even leading to cell death or uncontrolled differentiation (Kropp, Massai, et Zweigerdt 2017) **[5].** Scientific evidence demonstrated that agglomerates with a diameter larger than 400 µm are critical for cell viability (Li et al. 2018) **[6].**

To maintain the agglomerates within a reasonable size range, a high shear stress shall be applied, which may affect the cells.

So far, significant efforts have been invested to provide optimized solutions allowing maximizing Stem Cell density in culture using standard bioreactors, such as wave bags, spinner flasks, vertical wheel bioreactors, or stirred tanks.

Continuous perfusion of the cell culture medium, with monitoring of the metabolic species in the bioreactor, permitted to maximize cell density of hPSCs up to 35 10⁶ cells/mL (Manstein et al. 2021) **[7],** but has not been demonstrated beyond a 500 mL volume reactor.

A possible option to protect cells from shear stress, further preventing cells from forming large agglomerates, is cell encapsulation.

Li et al. demonstrated culture of hPSC at high density of 5 10⁸ cells/mL when encapsulating cells in 400 µm diameter alginate tubes **[6].**

Nevertheless, these tubes are fragile structures, further characterized by poor stability on the long term.

As a consequence, these tubes cannot be easily handled, which limits production scale and process reliability.

In principle, cell encapsulation in hydrogel spherical microcapsules has proven effective in protecting cells from shear stress.

Hydrogel microcapsules also constitute ideal carriers that can be easily handled in a process.

However, large scale production of these hydrogel microcapsules still represents a challenge.

Also, the encapsulation process may add stress to the cells, and must be completed within a reasonable timeframe.

For example, to encapsulate cells to be inoculated into a large 200 L reactor, it is required to encapsulate cells at a rate of about 1 to 10 L/min in order to keep the overall timing for whole process steps (i.e., including capsule formation and stabilization) below three hours.

Microfluidics can be used to form very uniform microcapsules and core-shell microcapsules for Stem Cell encapsulation (Nazari et al. 2022) **[8].**

Nevertheless, this is limited to low flow rates, as shown in the experiment described in Langer et al., where about 24 microcapsules of 290 µm diameter were produced per second, this corresponding to a flow rate of only 0,02 mL.min⁻¹ (Langer et Joensson 2020) **[9].**

Faster techniques have been reported, such as spray nozzles that can produce 3000 microcapsules of 410 µm diameter per second, corresponding to a flow rate of 12 mL.min⁻¹ (Cohen et al. 2023) **[10].**

To reach a flow rate of 1 to 10 L/min, parallelization of thousands of microchannels or nozzles are required, which is not convenient in an industrial setting.

In the light of the above, it is an object of the invention to provide a method of high-density cell culture allowing reliably implementing cell culture on a large scale, with reduced costs.

According to the invention, a method of high-density cell culture comprises at least the following steps:
S1: obtain hydrogel microcapsules through membrane emulsification;
S2: encapsulate a selected number of cells per each obtained hydrogel microcapsule, and
S3: culture the encapsulated cells in a cell culture medium.

The present invention provides a method of high-density cell culture.

The method comprises a step of obtaining hydrogel microcapsules through membrane emulsification.

The method further comprises a step of encapsulating a selected number of cells per each obtained hydrogel microcapsule.

The method further comprises a step of culturing the encapsulated cells in a cell culture medium.

Membrane emulsification allows obtaining uniformly-sized hydrogel microdroplets and, in turn, hydrogel microcapsules, at larger flow rates compared to other microfluidic technologies.

For instance, formation of microdroplets using membrane emulsification system were reported at flow rates up to 5730 L.m⁻².h⁻¹, corresponding to 0,3 L.min⁻¹ when using a 100 mm long and 10 mm diameter tubular membrane emulsification device (Holdich et al. 2020) **[11].**

As an example, the membrane emulsification system (commercially available system) is capable of producing droplets or hydrogel microcapsules at a rate of up to 1500 kg/h, and is compatible with good manufacturing practices (known as "GMP").

Preferably, the method further comprises a step of differentiating the cultured encapsulated cells in a cell differentiation medium.

Cell differentiation is carried out while maintaining the expanded cells encapsulated in the hydrogel microcapsules.

Preferably, the method further comprises a step of decapsulating the hydrogel microcapsules.

The invention is based on the basic idea that, by culturing (and, optionally, differentiating) cells, in particular Stem Cells or Stem-Cell-Derived cells, encapsulated in hydrogel microcapsules obtained through membrane emulsification, it is possible to obtain high quality cells, in particular Stem Cells or Stem-Cell-Derived cells, on large scale and with reduced costs.

It is important that the used hydrogel shows a sufficient level of stability over the entire process.

For example, for cells having a 40- to 60-hour doubling time such as megakaryocytes progenitor cells, it is important that the used hydrogel remains stable for 40 to 60 days in cell culture medium.

The hydrogel microcapsules can be made of a polysaccharide-based biocompatible hydrogel.

This provides a natural environment for the cells to grow.

For example, said polysaccharide-based biocompatible hydrogel may comprise one or more among alginate, chitosan, starch, carrageenan, pectin, methylcellulose, cellulose sulfate, carboxymethylcellulose, or other cellulose-based compounds, or dextran, dextran sulfate, or other dextran-based compounds, or hyaluronic acid.

Additionally or alternatively, the hydrogel microcapsules can be made of a polymer-based biocompatible hydrogel.

For example, said polymer-based biocompatible hydrogel may comprise one or more among poly vinyl alcohol (PVA), pol poly (ethylene glycol) (PEG), poly (ethylene oxide) (PEO), poly (2-hydroxyethyl methacrylate) (PHEMA), poly (acrylic acid) (PAA), poly (acrylamide) (PAAm), or derivatives thereof.

The microcapsules are stabilized with a polyanion/polycation complex.

Advantageously, in the method of the invention, the hydrogel microcapsules can be alginate gel microcapsules stabilized with a polyanion/polycation complex.

Alginate gel is known for cell encapsulation applications.

Nevertheless, as mentioned, alginate gel cross-linked, e.g., using calcium ions (Ca2+) shows a poor stability over long term.

On the other hand, polyanion/polycation complexes are highly stable.

The use of a polyanion/polycation complex as stabilizer has proven effective for the purpose of obtaining hydrogel microcapsules showing a high level of stability, even on the long term.

Advantageously, the obtained microcapsules can be uniformly-sized spherical microcapsules having a diameter between 50 µm and 800 µm, preferably between 150 µm and 400 µm.

Preferably the cells are Stem Cells or Stem-Cell-Derived cells.

Preferably, the Stem Cells are Pluripotent Stem Cells (PSCs), in particular Human Pluripotent Stem Cells (hPSCs), or Adult Stem Cells (ASCs).

The encapsulation step comprises encapsulating at least one cell per each hydrogel microcapsule.

To facilitate cell growth, cells may be seeded at a higher density, e.g., with 100 to 1,000 cells per each hydrogel microcapsule.

For example, and more preferably, 1,000 megakaryocyte progenitor cells can be encapsulated per microcapsule, preferably a spherical capsule having a diameter of 400 µm.

The cells are then cultured until each microcapsule is filled with cells.

For example, for a 400 µm diameter microcapsule, this corresponds to a number of up to 64,000 cells per capsule for cells of 10 µm in diameter, or up to 8,000 cells per capsule for cells of 20 µm in diameter.

The cell culture step may be carried out in a bioreactor.

Said bioreactor can be a fixed-bed bioreactor.

Alternatively, said bioreactor can be a packed-bed bioreactor.

As a further alternative, said bioreactor can be a rotating bed bioreactor.

Preferably, the bioreactor allows for a solid/liquid ratio between 15% and 50%.

More preferably, the bioreactor allows for a solid/liquid ratio of 50%.

In principle, to maximize cell density, the solid/liquid ratio in the bioreactor shall be maximized.

In stirred-tank bioreactors, the maximum solid/liquid ratio is only up to about 15%, thus requiring 6 unit volumes of cell culture medium per unit volume of microcapsules [10].

For a 400 µm microcapsule, having a volume of 3 10⁻⁵ mL and filled with 64,000 cells, this corresponds to an effective cell density of 2.78 10⁸ cells/mL in the bioreactor.

This determines a significant increase in the overall costs, thus rendering the process less cost-effective.

Conversely, fixed-bed, packed-bed, or rotating-bed bioreactors, in which hydrogel beads can be retained, allow for a much higher solid/liquid ratio of up to 50%, where only one unit volume of cell culture medium per unit volume of microcapsules is required.

For a 400 µm microcapsule, having a volume of 3 10⁻⁵ mL and filled with 64,000 cells, this corresponds to an effective cell density of 9.55 10⁸ cells/mL in the bioreactor.

This option is thus deemed preferable for the purpose of maximizing cell density.

The cell culture medium may comprise a growth factor cocktail and a basal medium.

In particular, the growth factor cocktail may comprise at least one of Thrombopoietin (TPO), TPO receptor agonist, stem cell factor (SCF), interleukin 3 (IL-3), interleukin 6 (IL-6), interleukin 9 (IL-9) or interleukin 11 (IL-11).

Alternatively, the growth factor cocktail may comprise at least one of granulocyte-macrophage colony-stimulating factor (GM-CSF), delta-like canonical Notch ligand 4 (DLL4), Activin A, NOG, vascular endothelial growth factor (VEGF), tumor necrosis factor-α (TNF-α), interleukin 2 (IL-2), fibroblast growth factor (FGF), interleukin 1β (IL1β), and epidermal growth factor (EGF).

The latter option is in particular advantageous for iPSCs culturing.

Stem Cells or Stem-Cell-Derived cells can be differentiated towards mature cells.

Cell differentiation can be induced by changing the cell culture medium and cocktail of growth factor(s) to drive cells into the differentiation path.

Alternatively, cell differentiation can be induced by removing a differentiation blocker.

As a further alternative, cell differentiation can be induced by using the same cell culture medium for spontaneous differentiation

Differentiation blocker may comprise induced-specific gene expression to ensure the proliferation of the cells and hindering the differentiation.

In particular, the cell differentiation medium may comprise a growth factor cocktail and a basal medium (e.g., Iscove's Modified Dulbecco's Medium).

The method may further include a step of freezing the encapsulated cells for cryopreservation.

Here, the hydrogel microcapsules serve as cryoprotectant for the cells (Egorikhina et al. 2024) **[12].**

In particular, the properties of the hydrogel help preserving the cells during freezing, by confining the ice crystal growth, decreasing the freezing point and minimizing the osmotic shock (Zhang et al. 2018) **[13].**

The encapsulated cells can thus be safely and reliably cryopreserved, without risk of damage.

The freezing step for cryopreservation may be carried out right after cell encapsulation.

Alternatively, said freezing step may be carried out after cell culture.

This allows cryopreserving cells at a much higher density.

Eventually, said freezing step may be carried out after cell differentiation.

It is important that the hydrogel microcapsules can be decapsulated at the end of the process using a compound that is not harmful for the cells.

For instance, in the method of the invention, decapsulation of the hydrogel microcapsules may be conveniently implemented by using an enzyme.

Preferably, the chosen enzyme is able to target and digest the polyanion and/or the polycation to destabilize the complex.

For example, and preferably, said enzyme may comprise one of alginase or alginate lyase to target alginate, or cellulase to target cellulose sulfate, or dextranase to target dextran, or other polysacharride lyase.

The invention further relates to Human Pluripotent Stem Cells (hPSCs) obtained through the above-described method, in particular for use in medicine.

Still further, the invention relates to hPSCs as defined above, in particular for use in a method of *in vitro* generation of mature megakaryocytes (MKs).

Further details and advantages of the invention will now be disclosed in connection with the drawings, where:
- **Fig. 1**: is a block diagram schematically illustrating the steps of a method of high-density cell culture according to an embodiment of the invention;
- **Fig. 2**: is a schematic representation of the formation of microdroplets using membrane emulsification;
- **Fig. 3**: is a schematic, cross-section representation of stacked flat sheet membranes for membrane emulsification;
- **Fig. 4**: is a schematic, cross-section representation of a tubular membrane with continuous phase flowing at the center;
- **Fig. 5**: is a schematic, cross-section representation of a tubular membrane with continuous phase flowing at the center;
- **Fig. 6**: is a schematic, cross-section representation of a tubular membrane with internal tubular spacer;
- **Fig. 7**: is a diagram schematically illustrating hydrogel microcapsules in a stirred-tank bioreactor;
- **Fig. 8**: is a diagram schematically illustrating hydrogel microcapsules in. **a.** a rotating bed bioreactor; **b.** a packed-bed bioreactor; **c.** a fixed-bed bioreactor.

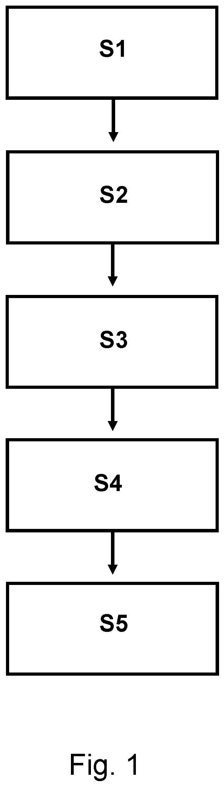

**Fig. 1** schematically shows the steps of a method of high-density cell culture according to an embodiment of the invention.

The method includes at least the following steps:
S1: obtain hydrogel microcapsules through membrane emulsification;
S2: encapsulate a selected number of cells per each obtained hydrogel microcapsule, and
S3: culture the encapsulated cells in a cell culture medium.

In the present embodiment, as shown in **Fig. 1****,** the method further comprises the following steps:
S4: differentiate the cultured encapsulated cells in a differentiation medium, and
S5: decapsulate the hydrogel microcapsules.

In the present embodiment, the cells are Stem Cells or Stem Cell-Derived cells.

In the present embodiment, the Stem Cells are Pluripotent Stem Cells (PSCs), in particular Human Pluripotent Stem Cells (hPSCs), or Adult Stem Cells (ASCs).

The formation of the hydrogel microcapsules occurs in two steps: formation of a two-phase emulsion using membrane emulsification, followed by stabilization of the formed microdroplets.

The two-phase emulsion can be an aqueous (dispersed phase) in oil (continuous phase) emulsion, or an aqueous two-phase emulsion.

The dispersed phase contains the cells.

The dispersed phase is pushed through a membrane, into the continuous phase.

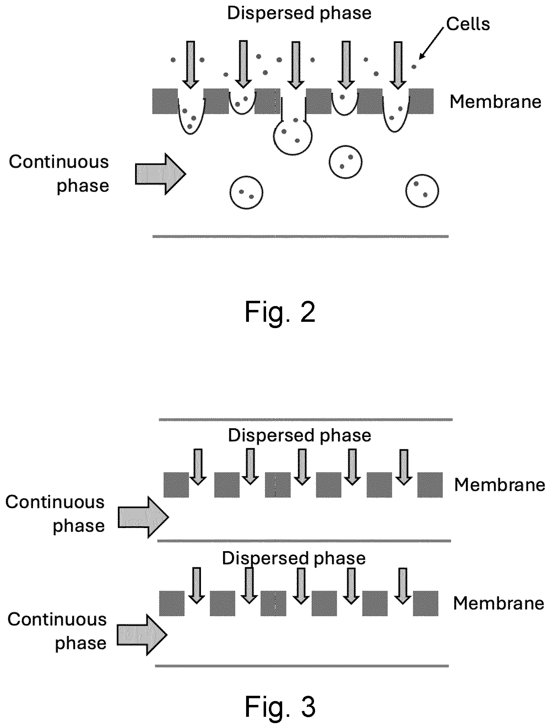

The continuous phase is flown perpendicularly to the membrane to induce shear stress that will permit to form the microcapsules, as shown in **Fig.2****.**

To maximize the flow rate for microcapsules formation, it is required to maximize the total membrane surface.

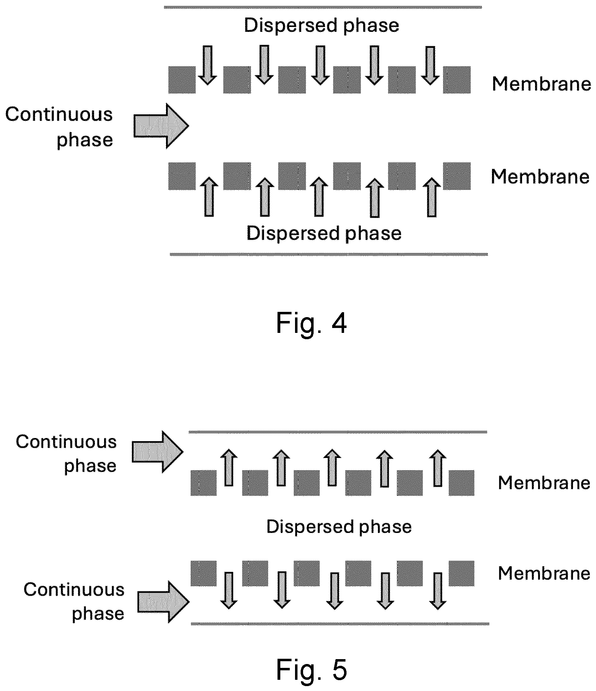

To this end, different membrane configurations can be used, such as a stacking of several flat sheet membranes spaced by separators, as shown in **Fig. 3****,** or a tubular membrane, as in the case of **Figs. 4** to **6****.**

The microcapsules may contain one or more biocompatible hydrogels, e.g., polysaccharides-based hydrogels, providing a natural environment for the cells to grow.

Preferably, said one or more polysaccharides-based hydrogel may comprise one or more among alginate, chitosan, starch, carrageenan, pectin, methylcellulose, cellulose sulfate, carboxymethylcellulose, or other cellulose-based compounds, dextran, dextran sulfate, or other dextran-based compounds, or hyaluronic acid.

Additionally or alternatively, the microcapsules may contain one or more polymer-based biocompatible hydrogels.

Preferably, said one or more polymer-based biocompatible hydrogels may comprise one or more among poly vinyl alcohol (PVA), pol poly (ethylene glycol) (PEG), poly (ethylene oxide) (PEO), poly (2-hydroxyethyl methacrylate) (PHEMA), poly (acrylic acid) (PAA), poly (acrylamide) (PAAm), or derivatives thereof.

The microcapsules may contain a mix of different hydrogels.

The microcapsules are then stabilized using cross-linking agents, or using compounds to initiate cross-linking of the hydrogels and form stable hydrogel microcapsules.

Preferably, the microcapsules are stabilized using a polyanion/polycation complex, e.g., alginate/chitosan, or alginate/polylysine, or alginate/protamine, or cellulose sulfate/oly(diallyldimethylammonium chloride) (PDADMAC), or poly(sodium-4-styrenesulfonate) (PSS)/Poly(allylamine hydrochloride) (PAH).

Other examples of polyanions include: poly(acrylic acid) (PAA), carboxymethylcellulose, dextran sulfate, polyanionic cellulose, or hyaluronic acid.

Other examples of polycations include: poly(diallyldimethylammonium chloride) (PDADMAC), polyethylenimine, polyamines, or starch.

In this case, the microcapsules contain at least a polyanion and/or a polycation, and the complementary element, a polycation or a polyanion, respectively, is added to the continuous phase of the emulsion to stabilize the microcapsule.

In the present embodiment, the hydrogel microcapsules are alginate gel microcapsules stabilized with a polyanion/polycation complex.

In general, Alginate gel microcapsules have a reduced stability on the long term.

By stabilizing Alginate gel microcapsules with a polyanion/polycation complex (which, on the other hand, is highly stable), hydrogel capsules can be obtained, showing a high level of stability over the entire process, e.g., over a period of several weeks.

As an example, when cells with a 40- to 60-hours doubling time are cultured, the used hydrogel shall remain stable for at least 40 to 60 days.

The hydrogel microcapsules provide protection for the cells against shear stress.

This is particularly advantageous when Stem Cells or Stem-Cell-Derived cells are cultured, since these cells are highly sensitive to shear stress.

Also, hydrogel microcapsules allow preventing the cultured cells from forming large agglomerates, which may lead to cell damage or even death.

Still further, by using hydrogel microcapsules obtained through membrane emulsification, it is possible to obtain high quality cells on a large scale, and with reduced costs.

Accordingly, a clinically-relevant amount of cells can be obtained, with reduced production costs.

In the present embodiment, the hydrogel microcapsules are uniformly-sized spherical microcapsules having a diameter between 50 µm and 800 µm, preferably between 150 µm and 400 µm.

In the present embodiment, the encapsulation step comprises encapsulating at least one cell per each hydrogel microcapsule.

To facilitate cell growth, the cells may be seeded at a higher density, e.g., with 100 to 1,000 cells per each hydrogel microcapsule.

For example, and more preferably, 1,000 megakaryocyte progenitor cells can be encapsulated per microcapsule, preferably a spherical capsule having a diameter of 400 µm.

The cells are then cultured until each microcapsule is filled with cells.

For example, for a 400 µm diameter microcapsule, this corresponds to a number of up to 64,000 cells per capsule for cells of 10 µm in diameter, or up to 8,000 cells per capsule for cells of 20 µm in diameter.

Then, cells are cultured until the entire microcapsule is filled with cells, corresponding to a number of approximately 64,000 cells per capsule, for a 400 µm diameter microcapsule.

In order to maximize cell density, the solid/liquid ratio in the bioreactor shall be maximized.

Conveniently, in the present embodiment, cell culture is carried out in a fixed-bed bioreactor, a packed-bed bioreactor, or a rotating bed reactor, allowing for a solid/liquid ratio between 15% and 50%, preferably a solid/liquid ratio of 50%.

This allows maximizing cell density.

As a consequence, the overall production costs can be minimized, since a reduced volume of culture medium is required, e.g., when compared to stirred-tank bioreactors allowing for a maximum solid/liquid ratio of about 15%.

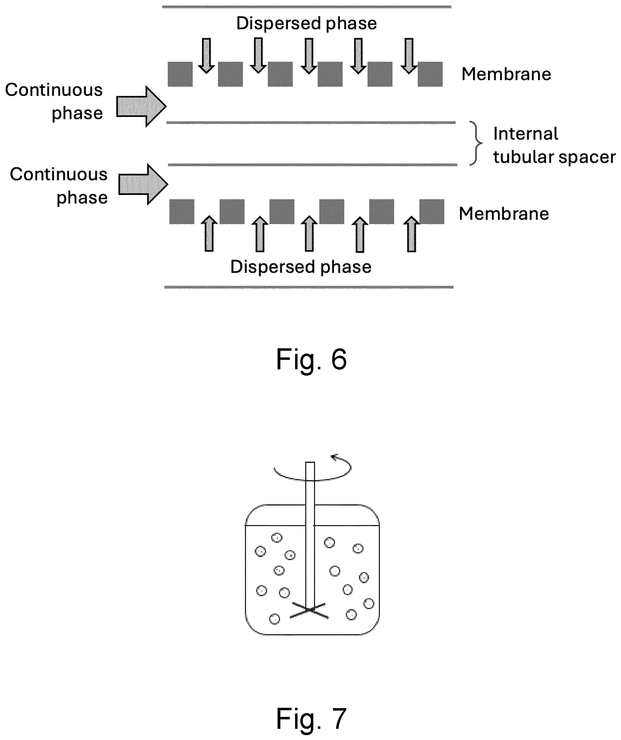

In stirred-tank bioreactors **(****Fig. 7****),** the maximum solid/liquid ratio is only up to about 15%, thus requiring 6 unit volumes of cell culture medium per unit volume of microcapsules **[10].**

For a 400 µm microcapsule, having a volume of 3 10⁻⁵ mL and filled with 64,000 cells, this corresponds to an effective cell density of 2.78 10⁸ cells/mL in the bioreactor.

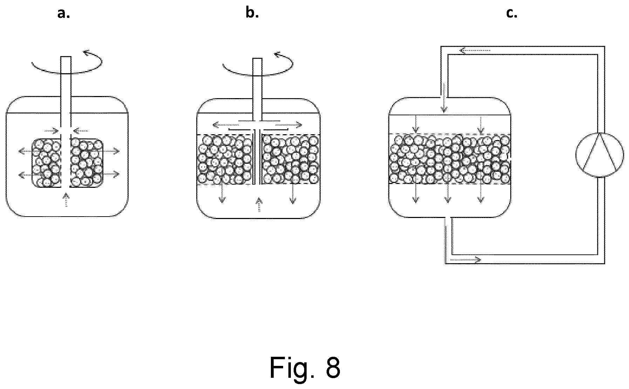

Conversely, rotating bed bioreactors (**Fig. 8a**), packed-bag bioreactors (**Fig. 8b**), or fixed-bag bioreactors (**Fig. 8c**), in which hydrogel beads can be retained, allow for a much higher solid/liquid ratio of up to 50%, where only one unit volume of cell culture medium per unit volume of microcapsules is required.

For a 400 µm microcapsule, having a volume of 3 10⁻⁵ mL and filled with 64,000 cells, this corresponds to an effective cell density of 9.55 10⁸ cells/mL in the bioreactor.

This option is thus deemed preferable for the purpose of maximizing cell density.

In the present embodiment, the cell culture medium comprises a growth factor cocktail and a basal medium.

In particular, said growth factor cocktail may comprise at least one of Thrombopoietin (TPO), TPO receptor agonist, stem cell factor (SCF), interleukin 3 (IL-3), interleukin 6 (IL-6), interleukin 9 (IL-9) or interleukin 11 (IL-11).

Alternatively, said growth factor cocktail may comprise at least one of granulocyte-macrophage colony-stimulating factor (GM-CSF), delta-like canonical Notch ligand 4 (DLL4), Activin A, NOG, vascular endothelial growth factor (VEGF), tumor necrosis factor-α (TNF-α), interleukin 2 (IL-2), fibroblast growth factor (FGF), interleukin 1β (IL1β), and epidermal growth factor (EGF).

Stem Cells or Stem-Cell-Derived cells can be differentiated towards mature cells.

In particular, in the method of the invention, differentiation can be induced by:
changing the cell culture medium and cocktail growth factor(s) to drive cells into the differentiation path, or
removing a differentiation blocker, or
using the cell culture medium for spontaneous differentiation.

In the present embodiment, decapsulation of the hydrogel microcapsules is carried out by using an enzyme, preferably an enzyme that is able to target and digest the polyanion and/ the polycation to destabilize the complex.

For instance, the enzyme may comprise one of:
alginase or alginate lyase to target alginate, or
cellulase to target cellulose sulfate, or
or dextranase to target dextran, or
or other kind of polysacharride lyase.

This allows decapsulating the microcapsules without any risk of harming the cells.

The method may further comprise a step of freezing the encapsulated cells for cryopreservation.

In particular, the properties of hydrogels help preserving the cells during freezing by confining the ice crystal growth, decreasing the freezing point and minimizing the osmotic shock [13].

The freezing step may be performed right after cell encapsulation, after cell expansion (thus permitting to cryopreserve cells at a higher density), or even after cell differentiation.

The invention further relates to Human Pluripotent Stem Cells (hPSCs) obtained through the above-described method, in particular for use in medicine.

The invention further relates to hPSCs as defined above, in particular for use in a method of *in vitro* generation of mature megakaryocytes.

### References

- S1: Method step
- S2: Method step
- S3: Method step
- S4: Method step
- S5: Method step

### Bibliography

**[1]** Grompe 2002
**[2]** Prochazkova et al. 2015
**[3]** Passier et Mummery 2003
**[4]** Sugimoto et al. 2022
**[5]** Kropp, Massai, et Zweigerdt 2017
**[6]** Li et al. 2018
**[7]** Manstein et al. 2021
**[8]** Nazari et al. 2022
**[9]** Langer et Joensson 2020
**[10]** Cohen et al. 2023
**[11]** Holdich et al. 2020
**[12]** Egorikhina et al. 2024
**[13]** Zhang et al. 2018

## Claims

1. A method of high-density cell culture comprising at least the following steps:
S1: obtain hydrogel microcapsules through membrane emulsification;
S2: encapsulate a selected number of cells per each obtained hydrogel microcapsule, and
S3: culture the encapsulated cells in a cell culture medium.

2. The method of claim 1,
**characterized in that**
the method further comprises the following step:
S4: differentiate the cultured encapsulated cells in a differentiation medium.

3. The method of claim 1 or 2,
**characterized in that**
the method further comprises the following step:
S5: decapsulate the hydrogel microcapsules.

4. The method of any of the preceding claims,
**characterized in that**
the hydrogel microcapsules are made of a polysaccharide-based biocompatible hydrogel, preferably wherein said polysaccharide-based biocompatible hydrogel comprises one or more among alginate, chitosan, starch, carrageenan, pectin, methylcellulose, cellulose sulfate, carboxymethylcellulose, or other cellulose-based compounds, dextran, dextran sulfate, or other dextran-based compounds, or hyaluronic acid, and/or
the hydrogel microcapsules are made of a polymer-based biocompatible hydrogel, preferably wherein said polymer-based biocompatible hydrogel comprises one or more among, poly vinyl alcohol (PVA), pol poly (ethylene glycol) (PEG), poly (ethylene oxide) (PEO), poly (2-hydroxyethyl methacrylate) (PHEMA), poly (acrylic acid) (PAA), poly (acrylamide) (PAAm), or derivatives thereof,
wherein the microcapsules are stabilized with a polyanion/polycation complex.

5. The method of claim 4,
**characterized in that**
the hydrogel microcapsules are alginate gel microcapsules stabilized with a polyanion/polycation complex.

6. The method of any of the preceding claims,
**characterized in that**
the obtained hydrogel microcapsules are uniformly-sized spherical microcapsules having a diameter between 50 µm and 800 µm, preferably between 150 µm and 400 µm.

7. The method of any of the preceding claims,
**characterized in that**
the cells are Stem Cells or Stem Cell-Derived cells.

8. The method of claim 7,
**characterized in that**
the stem cells are Pluripotent Stem Cells (PSCs), in particular Human Pluripotent Stem Cells (hPSCs) or Adult Stem Cells (ASCs).

9. The method of any of the preceding claims,
**characterized in that**
the encapsulation step comprises encapsulating at least one cell per each hydrogel microcapsule, preferably 100 to 1,000 cells per each hydrogel microcapsule, more preferably 1,000 cells per each hydrogel microcapsule.

10. The method of any of the preceding claims,
**characterized in that**
cell culture is carried out in a bioreactor, preferably a fixed-bed bioreactor, a packed-bed bioreactor, or a rotating bed bioreactor,
preferably wherein said bioreactor allows for a solid/liquid ratio between 15% and 50%, more preferably a solid/liquid ratio of 50%.

11. The method of any of the preceding claims,
**characterized in that**
the cell culture medium comprises a growth factor cocktail and a basal medium,
preferably wherein said growth factor cocktail comprises:
at least one of Thrombopoietin (TPO), TPO receptor agonist, stem cell factor (SCF), interleukin 3 (IL-3), interleukin 6 (IL-6), interleukin 9 (IL-9), or interleukin 11 (IL-11), or
at least one of granulocyte-macrophage colony-stimulating factor (GM-CSF), delta-like canonical Notch ligand 4 (DLL4), Activin A, NOG, vascular endothelial growth factor (VEGF), tumor necrosis factor-α (TNF-α), interleukin 2 (IL-2), fibroblast growth factor (FGF), interleukin 1β (IL1β), and epidermal growth factor (EGF).

12. The method of any one of claims 2 to 11,
**characterized in that**
cell differentiation is induced by:
changing the cell culture medium and cocktail growth factor(s) to drive cells into the differentiation path, or
removing a differentiation blocker, or
using the cell culture medium for spontaneous differentiation.

13. The method of any of the preceding claims,
**characterized in that**
the method further comprises a step of freezing the encapsulated cells for cryopreservation,
wherein said freezing step is carried out:
after cell encapsulation, or
after cell culture, or
after cell differentiation.

14. The method of any of claims 3 to 13,
**characterized in that**
decapsulation of the hydrogel microcapsules is carried out by using an enzyme, preferably an enzyme that is able to target and digest the polyanion and/or the polycation to destabilize the complex,
preferably wherein said at least one enzyme comprises one of:
alginase or alginate lyase to target alginate, or
cellulase to target cellulose sulfate, or
or dextranase to target dextran, or
or other kind of polysacharride lyase.

15. Human Pluripotent Stem Cells (hPSCs) obtained through the method of any of claims 1 to 14, for use in medicine.

16. Human Pluripotent Stem Cells (hPSCs) of claim 15, for use in a method of *in vitro* generation of mature megakaryocytes.
